# EUROPEAN PATENT APPLICATION

(11) **EP 4 360 674 A1**
(43) Date of publication of application: **01.05.2024**
(21) Application number: 22204208.7
(22) Date of filing: 27.10.2022
(51) Int. Cl.: A61M 5/315, G01D 5/12

(54) **AUTOMATIC INJECTION DEVICE WITH ROTATION SENSOR**

(71) Applicant: Ypsomed AG, 3401 Burgdorf (CH)
(72) Inventor: Schenker, Susanne, 4912 Aarwangen (CH); Glauser, Oliver, 3008 Bern (CH)
(74) Representative: Kleiner, Stefan

(57) **Abstract**

The present invention relates to an automatic drive mechanism (2) for an autoinjector (1) for dispensing a liquid drug from a prefilled syringe (20). The automatic drive mechanism (2) comprises a housing (12, 56), an energy source (15), a rotating member (40) operatively connected to the energy source (15) and rotatable relative to the housing (11) by the energy source for the dispensing, wherein the rotating member (40) has a surface structure (44) in the longitudinal direction varying in circumferential direction. The drive (2) further includes a sensor with a sensing member (51) which is movable by the surface structure when the rotating member (40) is rotated by the energy source (15). Furthermore, the sensing member (51) is guided by a linear guide (25) to move in the longitudinal direction and wherein the sensor assembly further includes a switch (61) which can be actuated by the movement of the sensing member (51) in the longitudinal direction.

## Description

### FIELD OF THE INVENTION

The present invention relates to drug delivery devices or medicament delivery devices for injecting, delivering, administering, infusing or dispensing substances and/or liquids such as insulin, hormone preparations or vaccines. It departs from an automatic drive mechanism for an autoinjector for dispensing a liquid drug from a reservoir. The automatic drive mechanism comprises a housing, an energy source for automatically moving a dispensing member for the dispensing, a rotating member rotatable by the energy source and a sensor assembly adapted to sense a rotation of the rotating member.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with prefilled syringes. Autoinjectors usually comprise a body for housing the syringe as well as an automatic drive mechanism in order to move the plunger of the syringe upon actuation of the autoinjector. The drive mechanism of the autoinjector typically comprises a source of drive, such as a motor or strong spring for moving a transfer element, for example a rod, which acts on the plunger of the syringe. The prefilled syringe has a needle or cannula that is permanently attached, glued, or otherwise staked to a first end of a container or reservoir that is sealed at the opposing end by the plunger. Alternatively, the autoinjector may be used with a carpule including a septum at the first end, which is pierced only just prior to injection by a needle that is shipped separate from the carpule.

For safety and hygiene reasons it is desirable that the needle does not protrude from a housing of the autoinjector with the exception of the time when the needle is used for injection of a medicament. Thus, either the autoinjector moves the needle out of the housing for the injection and back into the housing after injection or device includes a needle cover or needle guard which may be moved to unsheathe the needle for injection and which may be moved back in a needle covering position after the injection.

The majority of autoinjectors are configured as single use devices which incorporate both the syringe and the drive mechanism in the same housing. Such devices are usually disposable for hygiene reasons.

Disposable autoinjectors comprising electronics require a source of energy which is usually in the form of a battery. However, in this case, the autoinjectors should not be disposed in the regular waste, but have to be subjected to special disposal or to recycling. To account for a need to handle and dispose the autoinjector parts differently two-part autoinjectors have been developed. Such autoinjectors comprise a part which includes the electronics and a battery and which can be separated and disposed of separately from the other part, for example the drive mechanism and/or a syringe unit of the autoinjector.

Electronic modules in the autoinjector may be needed in order to allow a monitoring of the autoinjector, for example, to proactively prevent false handling of the device and/or to keep track of doses applied. The electronic modules may generate data related to an instantaneous condition and/or use of the delivery device. Different arrangements for detecting a rotation of a rotating member of the dispensing mechanism are known in the art for this purpose.

WO 2017/097507 A1 discloses an autoinjector comprising a plunger driver connected to a drive spring for automatically dispensing the medication inside the syringe. During dispensing of the medication a mechanical switch is rotated and moved in relation to fixed activation walls such that the mechanical switch is pressed against the walls upon rotation. When the plunger driver starts rotating, the mechanical switch is also brought to rotation since it is arranged on a rotating PCB. A communication device determines information based on a number of revolutions of the plunger driver. The determined number of revolutions are then compared with a correct number of revolutions being predetermined.

WO 2019/040313 A1 discloses a manual injection pen including a dose detection system with a rotational sensor which comprises a movable pin. A dedicated coil spring is positioned between a pin flange and a dose button and biases the pin in the distal direction of projections. As a flange rotates during dose delivery, the pin and the dose button maintain their relative position, and a contact surface of the pin rides up over each surface of the projections against the biasing force of the coil spring. The pin thereby operates as a follower member which follows the contours of the projections and recesses. A micro switch is operated to detect axial movement of the pin in the proximal direction each time the pin rides over a projection. The rotational sensor thus detects angular movement of the manual dose setting member by counting the number of projections that trigger sensing element during dose delivery. A dose-dispense movement of the dose button generates a first signal. This requires the pin to be aligned with one of the axial projections. In order to determine a dose size the flange comprises a plurality of fine-graded projections with at least one step per dose unit.

WO 2021/099432 A1 discloses a manual injection pen with a sensor comprising a movable sensing member which is engaged by formations of a manual dose dial sleeve during rotation of the dial sleeve. The rotation of the dial sleeve in a first rotational direction relative to a housing causes successive formations to be urged against the sensing member such that the sensing member is repeatedly moved between an unactuated state and an actuated state.

Rotation detection systems known in the art often comprise a complex and bulky structure or/and are adapted for a manual dose dial mechanism.

### DESCRIPTION OF THE INVENTION

It is an objective of the invention to provide a reliable dose measuring system with a space-saving design for an autoinjector with an automatic drive mechanism.

This objective is achieved by the automatic drive mechanism, the autoinjector or the method according to the independent claims. Preferred embodiments are evident from the dependent claims.

The invention relates to an automatic drive mechanism for an autoinjector for dispensing a liquid drug from a prefilled container or syringe. According to the invention the automatic drive mechanism comprises a housing extending in a longitudinal direction and adapted to accommodate
- an energy source for automatically moving a dispensing member for the dispensing of the drug from the syringe;
- a rotating member operatively, directly or indirectly, connected to the energy source and rotatable relative to the housing by the energy source for the dispensing, wherein the rotating member has a surface structure in the longitudinal direction varying in a circumferential direction;
- a sensor assembly adapted to sense a rotation of the rotating member by sensing the varying surface structure, wherein the sensor assembly includes a sensing member which is movable by the surface structure when the rotating member is rotated by the energy source wherein and the sensor assembly preferably further includes sensor electronics configured to generate a sensor signal based on the moving sensing member

The sensing member is guided by a linear guide to move in the longitudinal direction and wherein the sensor assembly further includes a switch, preferably arranged axially and rotationally fixed with respect to the housing. The switch can be actuated by the movement of the sensing member in the longitudinal direction.

As the sensing member is guided by the linear guide it can move exclusively in the longitudinal direction when the sensing member is moved by the varying surface structure. The switch is thus actuated in a well-defined direction and therefore a reliable detection of rotation of the rotating member is provided. This in turn provides a reliable determination of use of the automatic drive mechanism and thus allows to determine a dispensed dose of drug.

The linear guide preferably provides a guidance allowing exclusively a movement of the sensing member in the longitudinal direction. The linear guide may include, for example, a guiding opening, a spline and nut or groove connection or a dovetail guide.

The sensor assembly includes a switch or sensor (mechanical, electromechanical, inductive, capacitive) to detect the axial movement of the sensing member when the sensing member is moved by the surface structure. The varying surface structure of the rotating member is preferably formed by a defined number of trigger elements circumferentially arranged. Such trigger elements may be, for example, elevations, depressions, holes, openings, recesses, cams or taps. Therefore, the sensing member is moved in the longitudinal direction at least one time per revolution by such a trigger element.

The rotating member includes at least one trigger element forcing the sensing member at least one time per revolution of the rotating member. Preferably, the rotating member includes a plurality of trigger elements in circumferential direction allowing for a position determination within one revolution as the sensing member is moved axially a plurality of times during one revolution of the rotating member.

The rotating member is rotated by the energy source during dispensing of the drug. A certain rotation angle or a certain number of revolution of the rotating member is therefore assigned to a specific amount of dispensed drug. Therefore, by sensing the rotation of the rotating member a dispensed dose can be determined. In this way, a full administration or a prematurely terminated dispensing can be detected. The generated monitoring data can be stored in the autoinjector or transmitted to an external device, for example, by a communication module of the autoinjector.

The automatic drive mechanism is for an autoinjector holding a prefilled syringe with an injection needle non-removable attached to a syringe barrel. The automatic drive mechanism includes an energy source for automatically drive the dispensing member. That means there is no user force required to move the dispensing member to dispense the drug from the syringe. The energy source may be, for example, a pretensioned spring or a pretensioned elastomeric or rubber element or a drive with pressurized gas.

Autoinjectors are usually adapted to inject the full dose with one stroke without the possibility for setting an intermediate dose. Preferably, the autoinjector is devoid of any dose setting capabilities.

The rotating member is rotated during dose dispensing. That is, the rotating member may be connected directly or indirectly via one or more intermediate member to the energy source and driven by the energy source. Non-limiting examples of the rotating member are a support or shaft for a drive spring, a drive sleeve, a clutch member or an indication member indicating dose numbers to the user. The rotating member is preferably not directly operable or movable by the user.

The sensor assembly is adapted to generate a sensor output signal based on a detected longitudinal movement of the sensing member relative to the housing. For this purpose, the sensor assembly includes sensor electronics and a switch or a position sensor sensing the position or/and movement of the sensing member. Apart from the sensing member the sensor assembly and in particular the sensor electronics are preferably arranged in a non-movable manner relative to the housing. The sensing member is adapted to follow a contour of the surface structure in a circumferential direction in the longitudinal direction. The varying surface structure with its trigger elements (examples mentioned above) causes the sensing member to move in the longitudinal direction. Such movements are sensed and are represented accordingly in a sensor output signal of the sensor assembly.

The sensor assembly further includes a switch and by preference a mechanical switch which can be actuated by the longitudinal movement of the sensing member. Hence, each time a trigger element of the surface structure of the rotating member moves the sensing member the switch is actuated and thus the ongoing rotation and/or the rotational position of the rotating member relative to the housing can be determined. The hub or amplitude of the sensing member movement is determined by a height or longitudinal extension of the trigger element (e. g. an elevations or extension) with respect to an indent or recess, and defined by a switch amplitude. Alternatively, the sensor assembly may include a capacitive or inductive sensor configured to detect the longitudinal movement of the sensing member.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the autoinjector carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

In a preferred embodiment the surface structure of the rotating member forms a continuous contour in a circumferential direction in the longitudinal direction. Preferably during a whole revolution of the rotating member the sensing member is adapted to be in contact with at least a portion of the surface and is adapted to follow the contour of the surface structure.

The contour (or outline) of the surface structure allows for a continuous path for the sensing member to be in contact with the surface structure in circumferential direction albeit the surface structure may comprise elevations, depressions or recesses. That means during rotation of the rotating member the sensing member keeps in contact continuously with the at least a portion or with the entire surface structure and runs along the contour and is able to sense continuously trigger elements such as elevations, depressions and/or recesses in the surface structure. As the rotation is sensed by movements of the sensing member in the longitudinal direction an immediate and reliable detection of a rotation of the rotating member is made possible.

The sensing member has preferably an elongated form extending in the longitudinal direction. This enables a reliable guiding of the sensing member by the linear guide. The sensing member may be, for example, a cylindrical-shaped member, a pin, a rod or a sleeve.

In a preferred embodiment the varying surface structure is formed by elevations and depressions which are shaped as circular ring sectors preferably alternatingly arranged in a circumferential direction. The circular ring sectors enable a reliable sensing of the elevations or depressions as a transition or passage from one ring sector to another can be well defined and because the sensing member can pass reliably through the sections during rotation of the rotating member. The number of longitudinal movements of the sensing member can be defined by the number of ring sectors with elevations or depressions. Thus, the resolution of the rotation sensor can be determined by choosing a specific number of ring sectors. The smaller the ring sectors the more precise the angular position can be determined.

The surface structure of the rotating member is in a preferred embodiment arranged on a proximal end face of the rotating member. The rotating member may have a cylindrical or sleeve-shaped form and the surface structure may be arranged on its proximal end face. Accordingly, the sensor assembly may be arranged proximally to the rotating member. Such an arrangement allows for compact design of the automatic drive mechanism.

Preferably, the energy source is a pretensioned spring member and a first end of the spring member is connected to the rotating member. The second end of the spring member is preferably fixedly hold relative to the housing. Thus, in an initial state or shipping state the pretensioned spring and the rotating member are held in place and upon activation or firing of the drive mechanism the spring member can release and thereby rotate the rotating member to dispense the drug.

The spring member may be, for example, a coil spring, torsion spring, compression spring or a tension spring.

In a preferred embodiment the rotating member is a spring support and the spring member is a torsion spring wrapped around a cylindrical portion of the spring support. Hence, the rotating member supports the torsion spring and is fixedly connected to one of the two ends of the torsion spring. The torsion spring wrapped around a (middle) portion of the support allows for a compact design. The spring support is preferably sleeve-shaped or cylindrical-shaped and the torsion spring is arranged around the sleeve or cylinder. The spring support may comprise a distal and proximal end wall defining the cylindrical portion for the torsion spring. Preferably, the surface structure with the varying surface structure is arranged on the distal or proximal wall on an end face. In this way, the rotating member provides for a support for the spring as well as a sensing surface for the rotational sensor assembly.

In a further preferred embodiment the automatic drive mechanism comprises or consists of a first part and a housing part. The sensor assembly and in particular the sensing member and the sensor electronics are arranged in the second part wherein the second part is separable from the first part.

The first part preferably includes a dispensing mechanism and in particular the energy source, the rotating member and the dispensing member. The first part is preferably devoid of any electronic parts.

Therefore, the sensor assembly with its electronic components can be separated from the rest of the autoinjector and thus allows for a separate disposal of the components after use. That means electronics of the sensor assembly can be disposed separately in a suitable electronic waste and mechanical components of the drive mechanism of the first part can be disposed in another suitable waste for non-electronic parts.

Correspondingly, the housing of the drive mechanism preferably comprises a first housing parts enclosing the components of the first part and a second housing part enclosing the electronic components of the second part.

The housing includes preferably a predetermined breaking line (or breaking point) defining a separation line between the first part (and first housing part) and the second part (and second housing part). The second housing part can thus easily be separated from the first housing part along the breaking line. The second part with its electronic components can thus be separated in a well-defined manner. The predetermined breaking line may be implemented in form of a weakened or perforated portion in the housing or by a releasable connection such as a snap-fit connection or a breakable form-fit connection.

By preference, the second part is connected and held to the first part by a lock member in an initial state or shipping state. The lock member is movable relative to the first part or relative to the second part from a locked position in which the first and second parts are held and locked together into an unlocked position in which the first and second parts are unlocked and can be separated. In the unlocked position the first and second parts preferably remain held together but are not locked together anymore such that the user can release the two parts from each other by a release movement. That means in the unlocked position the first and second parts are still prevented from fall apart.

Alternatively, a movement of the lock member into the unlocked position directly separates the two housing parts from each other.

The lock member can be moved, for example, shifted, rotated or screwed between the locked position and the unlocked position to lock or to unlock the second part from the first part. For example, after use of the autoinjector the user may move the lock member manually to unlock the second housing part. Alternatively, the lock member may be moved automatically by the energy source. Non-limiting examples of the lock member are a bolt, a rod, a latch member, a pawl or a hook.

Preferably, in the locked position a relative release movement between the first and second part and in particular between the first housing part and the second housing part is prevented by the lock member (for example by a positive locking or form-fit locking) and wherein in the unlocked position a relative release movement between the first and second part is enabled. This provides a safe and reliable locking and unlocking between the first and second housing parts. The release movement to separate the second part from the first part may be, for example, a rotational movement, a linear movement or a combination thereof.

In a preferred embodiment the rotating member is adapted to move the lock member from the locked position into the unlocked position by a rotation of the rotating member. Thus, the rotating member automatically rotated by the energy source may shift, lift, rotate or otherwise move the lock member out of the locked position and into the unlocked position thereby unlocking the second part from the first part. The lock member is preferably held by friction in the unlocked position such that it cannot be moved back into the locked position.

That means by rotation of the rotating member the lock member is moved into its unlocking position and the first and second parts can be physically separated, for example, upon a manual release movement by the user. Alternatively, the subsequent release movement may be driven by an energy source.

Optionally, the lock member (which is preferably arranged in the second part) may engage the rotating member and in particular the rotating member surface. The varying surface structure of the rotating member preferably cause the lock member to move in the longitudinal direction.

The lock member is preferably linearly guided by the first and/or second part such that the lock member is rotationally fixed relative to the first and/or second part. Therefore, the lock member is moved in the same manner as the sensing member providing a secure and reliable locking and unlocking of the first and second part.

The rotating member may, for example, move the lock member from the locked position into the unlocked position upon initial rotation, e. g. when the autoinjector is fired and the energy source starts to rotate the rotating member or if the rotating member was rotated a predefined amount of revolutions or a predefined rotation angle. Alternatively, the lock member may be moved by the rotating member after a predefined number of revolutions, for example, when force falls under a predefined limit.

The invention relates further to an autoinjector comprising the automatic drive mechanism with the sensor assembly as descripted above. The autoinjector preferably comprises the prefilled syringe including the injection needle permanently, and non-removably staked to a syringe body.

The prefilled syringe is preferably non-movable hold inside the housing of the autoinjector. In particular the syringe is axially and radially fixed relative to the housing.

In a preferred embodiment the autoinjector comprises a needle shield, in particular a needle protection sleeve. The protection sleeve is preferably movable relative to the housing between a covering position, in which the needle is covered by the needle protection sleeve and a retracted position, in which the needle is revealed and accessible from outside.

Further, the needle protection sleeve is preferably biased in distal direction by a needle sleeve spring ensuring that the needle protection sleeve does not move unintentionally into the retracted position. In a preferred embodiment the needle protection sleeve is moved from the covering position into the retracted position if the user presses the distal end onto the injection site. Such a retraction movement into the retracted position preferably triggers the start of an injection process thereby releasing the energy source, in particular a drive spring to start the injection.

Furthermore, the invention relates a method for sensing a rotation of a rotating member in an automatic drive of an autoinjector during dispensing of a dose of drug from a prefilled syringe in the autoinjector. The method includes the steps of
- providing a rotating member operatively connected to an energy source of the autoinjector, wherein the rotating member has a surface structure in a longitudinal direction varying in a circumferential direction;
- rotating, by the energy source, the rotating member relative to the housing for the dispensing;
- moving a sensing member in the longitudinal direction by the varying surface structure during rotation of the rotating member;
- actuating a switch by the movement in the longitudinal direction of the sensing member
- detecting a rotation of the rotating member based on the actuation of the switch.

### BRIEF DESCRIPTION OF THE DRAWINGS

The subject matter of the invention will be explained in more detail in the following text with reference to preferred exemplary embodiments which are illustrated in the attached drawings, in which:
Fig. 1 depicts a perspective view of an autoinjector with an automatic drive mechanism according to the invention;
Fig. 2 depicts a plane view of a contact surface structure of a rotating member;
Fig. 3 depicts a sectional view of the autoinjector of figure 1;
Fig. 4a depicts a sectional view of a proximal portion of the automatic drive mechanism with the second housing part and with a locking member in the locked position;
Fig. 4b depicts the drive mechanism with the locking member in the unlocked position;
Fig. 5a depicts the drive mechanism with a detection pin in an initial positon;
Fig. 5b depicts the drive mechanism with the detection pin in an actuated position.

The reference symbols used in the drawings, and their primary meanings, are listed in summary form in the list of designations. In principle, identical parts are provided with the same reference symbols in the figures.

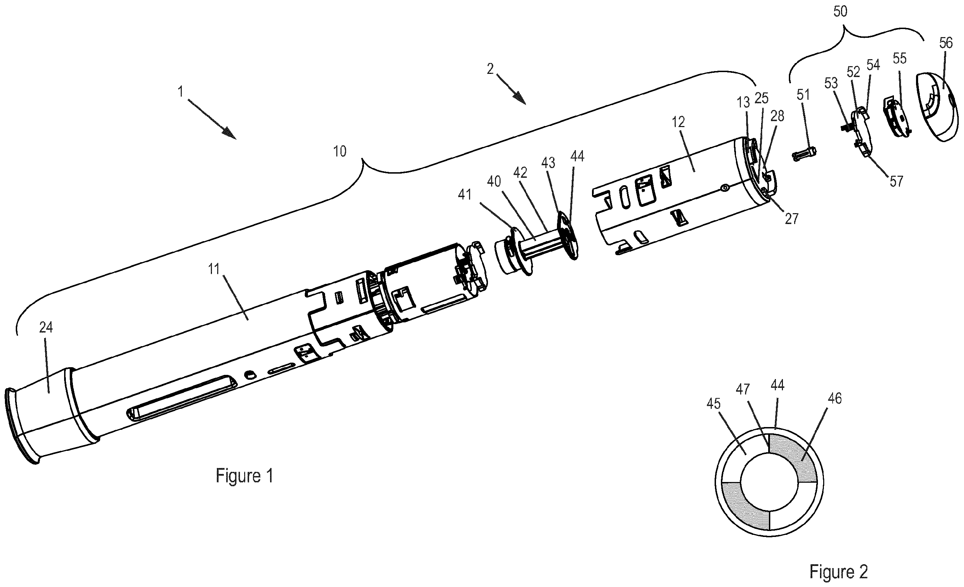

Figure 1 depicts a perspective and exploded view of an autoinjector 1 with an automatic drive mechanism 2 according to the invention.

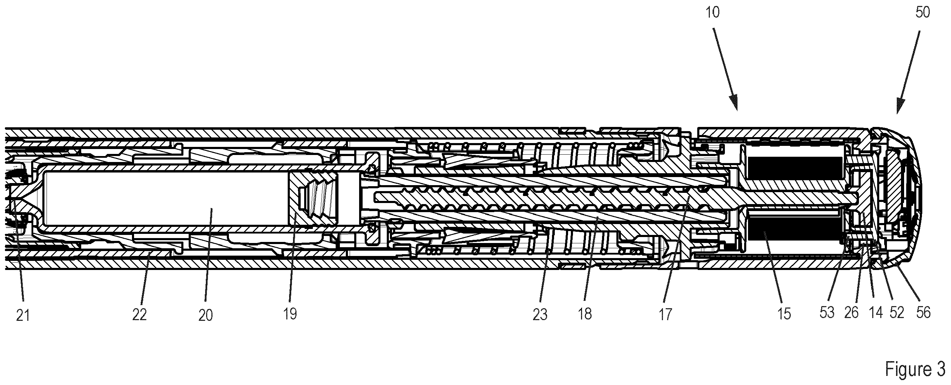

A distal end of the autoinjector 1 which includes the injection needle (covered by a cap 24 in the state shown in figure 1) is on the left hand side in figures 1 and 3. A proximal end of the autoinjector 1 is furthest away from the needle end and is on the right hand side in figures 1 and 3.

The drive mechanism 2 of the autoinjector 1 includes a first device part 10 and a second device part 50 which are descripted in detail below.

### First device part

The first device part 10 of the autoinjector 1 comprises a prefilled syringe 20 with a drug, an energy source for automatically dispense the drug from the syringe and a distal housing 11 and proximal housing 12 enclosing a first portion of the drive mechanism 2.

The most important features of the autoinjector 1 and in particular the dispensing mechanism are briefly described in the following. A detailed description of a dispensing mechanism of the shown autoinjector can be found in the patent application WO 2016/205961 A1 which is incorporated herein by reference.

The drive mechanism 2 comprises an energy source in form of a torsion spring 15, which is prestressed in an initial or unused state. The drive mechanism 2 further includes a spring support in form of a spring shaft spool 40 supporting the torsion spring. A first end of the torsion spring 15 is fixedly connected to the proximal housing 12 and the second end of the torsion spring 15 is connected to a cylindrical shaft portion 42 of the shaft spool 40. The latter is rotationally fixed to a threaded rod 17 as shown in figure 3. The threaded rod 17 in turn is in threaded connection with a plunger rod 18 and coaxially arranged inside the plunger rod 18.

In a distal portion of the autoinjector a needle protection sleeve 22 is coaxially arranged inside the distal housing 11. The protection sleeve 22 shields or covers a distal end portion of the injection needle 21 (figure 3) if the cap 24 is removed from the autoinjector 1. The needle protection sleeve 22 is movable relative to the distal and proximal housing 11, 12 between a covering position and a retracted position. The first device part 10 includes further a needle sleeve spring 23 biasing the needle protection sleeve 22 distally to ensure that the injection needle 21 is covered and that the protection sleeve 22 does not move unintentionally into the retracted position.

If the needle protection sleeve 22 is moved into the retracted position the prestressed torsion spring 15 is released and thus rotates the threaded rod 17. Due to the threaded connection and due to a linear guide the plunger rod 18 is non-rotationally moved in distal direction and thus it moves distally a piston 19 within the syringe 20. Therefore, the liquid drug inside the syringe 20 can be dispensed.

In figure 1 the spring shaft spool 40 can be seen in a perspective view. As shown the spring shaft spool 40 includes a distal flange 41 and a proximal flange 43 and a sleeve-shaped middle portion 42 connecting the two flanges 41, 43. As mentioned above the second end of the torsion spring 15 is connected to the middle portion 42. The proximal flange includes on its proximal end a surface structure or contact surface 44 including a pattern of circular ring sectors, see figure 2.

Figure 2 depicts a plane view of the contact surface 44. The ring sectors form either an elevation 46 or a depression 45 (or a recess or opening in the surface) and the elevations 46 and depressions 45 are alternatingly arranged in circumferential direction. The elevations 46 and depressions 45 are connected by ramps 47 which thus form a contact path in circumferential direction. In other words, the longitudinal extension of the proximal flange 43 varies due to the elevations 46 and depressions 45. The contact surface 44 is adapted to be contacted by a detection pin 51 as descripted with respect to the second device part 50.

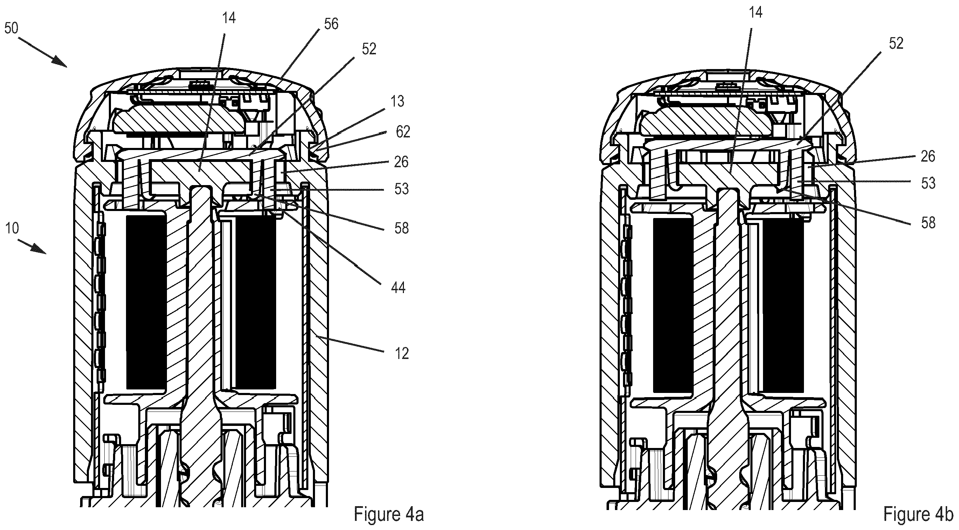

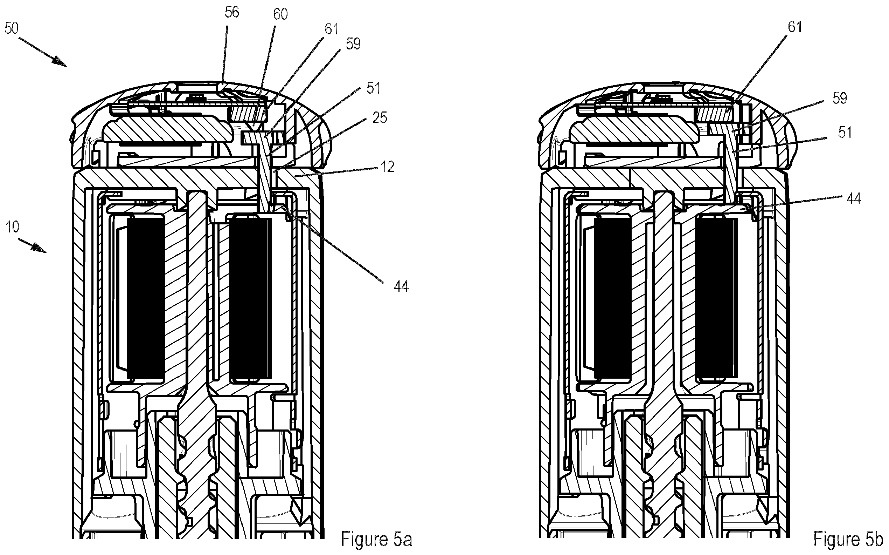

As shown in figure 1 and figures 5a, 5b the proximal housing 12 of the first device part 10 includes a proximal end wall 28 which comprises an opening 25 adapted to accommodate and guide the detection pin 51 of the second device part 50. Furthermore, as shown in figure 3 the end wall 28 includes additional through holes 26 (figure 3, 4a) to accommodate two contact pins 53 of a separation bolt 52 of the second device part 50.

### Second device part

The second device part 50 is intend to be separated from the first device part 10 by the user after use of the autoinjector 1. The second device part 50 includes electronic parts and is thus to be discarded separately form the first device part 10 which includes mainly plastic parts. The second device part 50 comprises a printed circuit board (PCB) 55, the separation bolt 52, the detection pin 51 and an end cover 56 enclosing the afore mentioned parts of the second device part 50.

The end cover 56 includes on its inside two projection 62 (see figure 4a) adapted to engage with two corresponding nuts 13 in axially protruding elements of the end wall 28 as it can be seen in figure 1. The engagement of the projections 62 with the nuts 13 forms a bayonet connection holding the second device part 50 on the proximal end of the first device part 10.

The PCB 55 is fixedly connected inside the end cover 56. The PCB 55 is connected to and supports a Bluetooth chip for wireless communication, LEDs, a mechanical switch 61 (best shown in figures 5a, 5b) and a battery which provides electrical energy to the PCB and its components.

As shown in figure 3, 4a and 4b the separation bolt 52 is arranged distally to the PCB 55. The separation bolt 52 is formed by a plat-shaped element with two distally extending contact pins 53. If the second device part 50 is mounted onto the proximal end of the first device part 10 the contact pins 53 extend through the holes 26 and are in contact with the contact surface 44 of the spring shaft spool 40 in an initial or delivery state.

As shown in figure 1 the separation bolt 52 further includes two side walls each forming a nut 54. The nut 54 is adapted to engage a spline on an inside of the end cover 56. The separation bolt 52 is thus guided for a movement in the longitudinal direction and rotation is prevented. The side walls form further a stop surface 57 engaging a counter stop surface 27 on an end cap element of the first device part if the second device part is mounted and locked to the first device part. The stop surfaces 57 abutting the counter stop surfaces 27 prevent the separation bolt 52 to be rotated relative to the end cover 56 and relative to the proximal housing part 12. That means this rotational lock provides a locking mechanism as described below to lock the second device part 50 to the first device part 10.

Besides the contact pins 53 the separation bolt 52 comprises hook elements 58 as shown in figure 3. The hook elements 58 limit a longitudinal movement of the separation bolt 52 in proximal direction relative to the first device part 10. That is, the hook elements 58 engage a plate element 14 of the proximal housing 12 thereby preventing further proximal movement of the separation bolt 52.

Furthermore, the separation bolt 52 includes a recess for the detection pin 51. The latter is cylindrical and comprises a contact portion 59 on its proximal end (see figures 5a, 5b). The contact portion 59 is adapted to contact a triangle-shaped actuation tip 60 of the mechanical switch 57. If the second device part 50 is mounted to the first device part 10 the distal end of the detection pin 51 extends through the opening 25 in the end wall 28. The distal end of the detection pin 51 is thereby in contact with the contact surface 44 of the spring shaft spool 40. The detection pin 51 is guided by a linear guide in form of a longitudinal slit or longitudinal nut in the end cover 56 such that the detection pin 51 is movable exclusively in the longitudinal direction.

### Function of the device

In an initial state or delivery state the second device part 50 is connected to the first device part 10 by the bayonet connection and locked by the stop surfaces 57, 27. That means the stop surfaces 57 of the separation bolt 52 are in contact with the counter stop surfaces 27 of the first device part end wall 28. The end cover 56 is thus locked and cannot be rotated relative to the first device part 10 and relative to the proximal housing 12. Due to this rotational lock the bayonet connection holding the second device part 50 and the first device part 10 together cannot be released.

Figure 4a shows the separation bolt 52 in its distal position or locked position. The distal ends of the separation bolt contact pins 53 are located in a depression 45 of the contact surface 44 of the spring shaft spool 40. In contrast, figure 4b shows the separation bolt 52 in its proximal or unlocked position allowing the second device part 50 to be removed from the first device part 10.

If the user presses the autoinjector 1 onto an injection site the needle protection sleeve 22 is moved proximally and thereby triggers the start of the injection. The prestressed torsion spring 15 is released and starts rotating the spring shaft spool 40 and the threaded rod 17. The latter moves the plunger rod 18 distally to dispense the drug.

If the spring shaft spool 40 starts to rotate the contact surface 44 with its elevations 46 and depression 45 starts to rotate relative to the contact pins 53 of the separation bolt 52 and relative to the detection pin 51. The means the contact pins 53 get into contact with a ramp 47 of the contact surface 44 when the spring shaft spool 40 starts rotating. If the spring shaft spool 40 further rotates the ramps 47 force the contact pins 53 and thus the whole separation bolt 52 in proximal direction. As the separation bolt 52 is linearly guided by the end cover 56 the separation bolt 52 is moved proximally unit the contact pins 53 reach the most proximal point (highest point) of the elevation 46 following the ramp 47. The separation bolt 52 is thus moved from its distal locked position into its proximal unlocked position as shown in figure 4b. The separation bolt hook elements 58 prevent further movement in proximal direction and additionally prevent a fall back in distal direction by friction as the hook elements 58 clamp on the plate element 14 the first device part 10.

With respect to figures 5a and 5b the function of the detection pin 51 is descripted in detail. The ramps 47 connecting the elevations 46 and depression 45 move also the detection pin 51 in the longitudinal direction. As the detection pin 51 is biased distally by the triangle-shaped and spring loaded actuation tip 60 of the mechanical switch 61 the detection pin 51 keeps in contact with the contact surface 44. Therefore, during rotation of the spring shaft spool 40 the detection pin 51 follows the elevations 46 and depressions 45 and is thereby moved alternatingly in distal and proximal direction in the longitudinal direction. Figure 5b shows a position of the contact surface 44 where the distal end of the detection pin 51 is onto an elevation 46 and thus the detection pin 51 was moved into a proximal end position. As depicted in figure 5b the contact portion 59 presses the actuation tip 60 proximally and thereby switches the switch 61. A controller in the PCB processes a signal generated by the actuated switch and detects the rotation of the spring spool shaft 40 and determines a number of revolutions based on the number of switching operations of the switch 61. Furthermore, based on the switch signal the controller determines if the dose has been fully dispensed or if the injection was terminated prematurely. The controller records the determined dispensed dose as well as the time and date of the injection. The generated injection data are send via Bluetooth to a user mobile phone or to any other external device.

If the injection is completed the autoinjector 1 has to be discarded. That is, the first device part 10 and second device part 50 have to be separated from each other in order to discard the electronics in the second device part 50 as electronic waste and separately from the first device part.

As the separation bolt 51 is in its unlocked position (shown in figure 4b). Hence, the stop surfaces 57 are axially separated from the counter stop surfaces 27. That means the end cover 56 can be rotated relative to the proximal housing 12 of the first device part 10. By a combination of a rotational and axial movement the projection 62 of the end cover 56 can be disengaged from the nut 13 of the proximal housing 12 and thereby the bayonet connection between the end cover 56 and the proximal housing 12 can be released. Subsequently, the user can remove the second device part 50 from the first device part 10.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

**LIST OF DESIGNATIONS**

| | | | |
|---|---|---|---|
| 1 | Autoinjector | 50 | second device part |
| 2 | drive mechanism | 51 | detection pin |
| | | 52 | separation bolt |
| 10 | first device part | 53 | contact pins |
| 11 | distal housing | 54 | nut |
| 12 | proximal housing | 55 | PCB |
| 13 | nut | 56 | end cover |
| 14 | plate element | 57 | stop surface |
| 15 | torsion spring | 58 | hook elements |
| 17 | threaded rod | 59 | contact portion |
| 18 | plunger rod | 60 | actuation tip |
| 19 | piston | 61 | mechanical switch |
| 20 | syringe | 62 | projection |
| 21 | needle | | |
| 22 | needle protection sleeve | | |
| 23 | needle sleeve spring | | |
| 24 | cap | | |
| 25 | opening | | |
| 26 | hole | | |
| 27 | counter stop surface | | |
| 28 | end wall | | |
| 40 | spring shaft spool | | |
| 41 | distal flange | | |
| 42 | middle portion | | |
| 43 | proximal flange | | |
| 44 | contact surface | | |
| 45 | depression | | |
| 46 | elevation | | |
| 47 | ramp | | |

## Claims

1. An automatic drive mechanism (2) for an autoinjector (1) for dispensing a liquid drug from a prefilled syringe (20), the automatic drive (2) comprising
- a housing (12, 56) extending in a longitudinal direction;
- an energy source (15) for automatically moving a dispensing member (18) for the dispensing;
- a rotating member (40) operatively connected to the energy source (15) and rotatable relative to the housing (11) by the energy source for the dispensing, wherein the rotating member (40) has a surface structure (44) in the longitudinal direction varying in a circumferential direction;
- a sensor assembly adapted to sense a rotation of the rotating member (40) and wherein the sensor assembly includes a sensing member (51) which is movable by the surface structure (44) when the rotating member (40) is rotated by the energy source (15) **characterized in that**
the sensing member (51) is guided by a linear guide (25) to move in the longitudinal direction and wherein the sensor assembly further includes a switch (61) which can be actuated by the movement of the sensing member (51) in the longitudinal direction.

2. Automatic drive mechanism (2) according to claim 1, wherein the surface structure (44) is a continuous contour in the circumferential direction in a longitudinal direction and wherein during a whole revolution of the rotating member (40) the sensing member (51) is adapted to be in contact with the surface structure (44) and is adapted to follow the contour of the surface structure (44).

3. Automatic drive mechanism (2) according to claim 1 or 2, wherein the sensing member (51) is an elongated member extending in the longitudinal direction.

4. Automatic drive mechanism (2) according to any of claims 1 to 3, wherein the varying surface structure (44) is formed by elevations (46) and depressions (45) shaped as circular ring sectors.

5. Automatic drive mechanism (2) according to any of claims 1 to 4, wherein the surface structure (44) is arranged on a proximal end face of the rotating member (40).

6. Automatic drive mechanism (2) according to any of claims 1 to 5, wherein the energy source (15) is a spring member and wherein one end of the spring member is connected to the rotating member (40).

7. Automatic drive mechanism (2) according to claim 6, wherein the rotating member (40) is a spring support and wherein the spring member is a torsion spring (15) wrapped around a cylindrical portion (42) of the spring support.

8. Automatic drive mechanism (2) according to any of claims 1 to 7, comprising a first part (10) and a second part (50) and wherein the energy source (15) and the rotating member (40) are arranged in the first part (10) and the sensor assembly is arranged in the second part (50) and wherein the second part (50) is separable from the first part (10).

9. Automatic drive mechanism (2) according to claim 8, wherein the housing (12, 56) includes a predetermined breaking line defining the first part (10) and the second part (50).

10. Automatic drive mechanism (2) according to claim 8 or 9, further comprising a lock member (52) movable relative to the first part (10) or the second part (50) from a locked position in which the first and second parts are locked together into an unlocked position in which the first and second parts can be separated.

11. Automatic drive mechanism (2) according to claim 10, wherein in the locked position a relative movement between the first and second part (10, 50) is prevented by the lock member (52) and wherein in the unlocked position a relative movement is enabled.

12. Automatic drive mechanism (2) according to claim 10 or 11, wherein the rotating member (40) is adapted to move the lock member (52) from the locked position into the unlocked position by a rotation of the rotating member (40).

13. Automatic drive mechanism (2) according to claim 12, wherein the varying surface structure (44) causes the lock member (52) to move in the longitudinal direction.

14. Autoinjector (1) adapted to receive a prefilled syringe (20) comprising an automatic drive according to claim 1 to 13.

15. Method for sensing a rotation of a rotating member (40) in an automatic drive mechanism (2) of an autoinjector (1) during dispensing of a dose of liquid drug, the method including the steps of
- providing a rotating member operatively connected to an energy source of the autoinjector, wherein the rotating member has a surface structure (44) in a longitudinal direction varying in a circumferential direction;
- rotating, by the energy source, the rotating member (40) relative to the housing for the dispensing;
- moving a sensing member (51) in the longitudinal direction by the varying surface structure (44) during rotation of the rotating member;
- actuating a switch (61) by the movement in the longitudinal direction of the sensing member
- detecting a rotation of the rotating member based on the actuation of the switch.
